# EUROPÄISCHE PATENTANMELDUNG

(11) **EP 2 628 791 A1**
(43) Veröffentlichungstag der Anmeldung: **21.08.2013**
(21) Anmeldenummer: 12156051.0
(22) Anmeldetag: 17.02.2012
(51) Int. Cl.: C12M 3/06, C12N 5/07

(54) **Perfusionsmodul für die Co-Kultur von Endothelzellen und Glattmuskelzellen zur Bestimmung pathosphysiologischer Veränderungen**

(71) Anmelder: Charité - Universitätsmedizin Berlin, 10117 Berlin (DE); Membrana GmbH, 42289 Wuppertal (DE)
(72) Erfinder: Die Erfindernennung liegt noch nicht vor
(74) Vertreter: Gulde Hengelhaupt Ziebig & Schneider

(57) **Zusammenfassung**

Die vorliegende Erfindung betrifft ein Perfusionsmodul zur Co-Kultur von Endothel- und Glattmuskelzellen, umfassend: i) eine oder mehrere Hohlfasern umfassend oder bestehend aus einer gas- und/oder flüssigkeitsdurchlässigen Membran mit einer durchschnittlichen Porengröße von 0,002 bis 1 µm, wobei mindestens Teile der luminalen Oberfläche der Hohlfasern mit Endothelzellen und mindestens Teile der abluminalen Oberfläche der Hohlfasern mit Glattmuskelzellen besiedelt sind; ii) ein Gehäuse zur Aufnahme der Hohlfasern; und iii) mindestens vier Zugänge, wobei die Hohlfasern, das Gehäuse und die Zugänge derart ausgestaltet und angeordnet sind, dass der extraluminale und der intraluminale Raum der Hohlfasern eines Perfusionsmoduls getrennt voneinander regel- und/oder steuerbar mit Flüssigkeit perfundierbar sind; sowie einen Bioreaktor umfassend ein solches Perfusionsmodul und ein Verfahren zur Bestimmung von Veränderungen im Sekretom von Endothelzellen und/oder Glattmuskelzellen.

## Beschreibung

Das Blutgefäßsystem sichert das Überleben des Organismus, indem es die Versorgung der Körperzellen erlaubt. Zum einen wird über das im Gefäßsystem zirkulierende Blut Sauerstoff zu den Zellen und Kohlendioxid in entgegengesetzter Richtung transportiert. Zum anderen werden Nährstoffe wie Fette, Zucker oder Eiweiße aus dem Verdauungstrakt in die einzelnen, zu versorgenden Gewebe transportiert, um dort je nach Bedarf verbraucht, weiterverarbeitet oder gespeichert zu werden. Die entstandenen Stoffwechsel- oder Abfallprodukte, zum Beispiel Harnstoff oder Harnsäure, werden dann in anderes Gewebe oder zu den Ausscheidungsorganen wie Nieren und Dickdarm transportiert. Außerdem verteilt das Blut auch Botenstoffe wie zum Beispiel Hormone, Zellen der Körperabwehr und Teile des Gerinnungssystems innerhalb des Körpers. Neben den physiologischen Aufgaben dient das Blutgefäß auch häufig dazu Wirkstoffe, beispielsweise Medikamente zur Bekämpfung bestimmter Erkrankungen, im Körper zu verteilen und an ihren Wirkort zu bringen.

Im Körper kleiden Endothelzellen die Innenflächen der Blutgefäße aus und bilden somit eine Barriere zwischen zirkulierendem Blut und dem umliegenden Gewebe. Auf der abluminalen Seite weisen Gefäße perivaskuläre Zellen, wie z. B. vaskuläre Glattmuskelzellen auf. Sowohl Nährstoffe als auch Wirkstoffe müssen diese Barriere überwinden, um zu dem zu versorgenden Gewebe zu gelangen. Daneben spielen Endothelzellen bei einer Reihe von Erkrankungen eine entscheidende Rolle und sind maßgeblich an physiologischen Prozessen, wie beispielsweise der Angiogenese beteiligt.

Um die Funktionsweise von Gefäßen und insbesondere Endothelzellen zu verstehen, sowie um Wirkstoffe zu identifizieren und zu entwickeln, die Eigenschaften von Endothelzellen oder Gefäßen beeinflussen bzw. diese ohne allzu große Beeinflussung passieren können, sind Zellkulturmodelle entwickelt worden, die meist eine horizontale, zweidimensionale Kultur von Endothelzellen oder ggf. eine Co-Kultur von Endothelzellen und perivaskulären Zellen umfassen.

Ein Problem dieser Zellkultursysteme ist, dass diese normalerweise nicht geeignet sind darzustellen, dass Endothelzellen in ihrer physiologischen Umgebung durch den Blutfluss einer ständigen laminaren Strömung und damit einem andauernden Scherstress ausgesetzt sind.

Ein weiteres Problem der bisher verfügbaren Zellkulturmodelle ist, dass diese mit relativ großen Zellkulturvolumina arbeiten und sich somit nicht für eine Parallelisierung und Miniaturisierung eignen.

Aufgabe der vorliegenden Erfindung ist es daher ein Zellkultursystem bereit zu stellen, welches eine Kultur von Endothelzellen unter möglichst physiologischen Bedingungen erlaubt und gleichzeitig parallelisier- und miniaturisierbar ist.

Die vorliegende Erfindung stellt ein Perfusionsmodul zur Co-Kultur von Endothel- und Glattmuskelzellen bereit, umfassend:
i) eine oder mehrere Hohlfasern umfassend oder bestehend aus einer gas- und/oder flüssigkeitsdurchlässigen Membran mit einer durchschnittlichen Porengröße von 0,002 bis 1 µm, wobei mindestens Teile der luminalen Oberfläche der Hohlfasern mit Endothelzellen und mindestens Teile der abluminalen Oberfläche der Hohlfasern mit Glattmuskelzellen besiedelt sind;
ii) ein Gehäuse zur Aufnahme der Hohlfasern; und
iii) mindestens vier Zugänge,
wobei die Hohlfasern, das Gehäuse und die Zugänge derart ausgestaltet und angeordnet sind, dass der extraluminale und der intraluminale Raum der Hohlfasern eines Perfusionsmoduls getrennt voneinander regel- und/oder steuerbar mit Flüssigkeit perfundierbar sind.

Das erfindungsgemäße Perfusionsmodul erlaubt die Co-Kultur von Endothelzellen und Glattmuskelzellen in einem räumlichen Aufbau, der dem Aufbau eines Blutgefäßes nahe kommt. Dabei können beide Zelltypen unter jeweils unterschiedlich regel- und/oder steuerbaren laminaren Flussbedingungen über einen langen Zeitraum von beispielsweise über 8 Tagen co-kultiviert werden. Dabei können die abluminalen perivaskulären Glattmuskelzellen und die Endothelzellen auf der luminalen Seite durch die Porenöffnungen der Hohlfasermembran miteinander kommunizieren, während ein direkter Zell-Zell-Kontakt zwischen diesen beiden Zelltypen im Wesentlichen ausgeschlossen bleibt. Die Hohlfasermembran übernimmt im erfindungsgemäßen Perfusionsmodell die Funktion der extrazellulären Matrix, die sich unter physiologischen Bedingungen in einem Gefäß zwischen Endothelzellschicht und Glattmuskelzellschicht befindet. Im erfindungsgemäßen Perfusionsmodul können also die physiologischen Bedingungen eines Blutgefäßes optimal nachgestellt werden. Das erfindungsgemäße Perfusionsmodul eignet sich beispielsweise zum modularen Aufbau von Bioreaktoren in dem eine Vielzahl von Perfusionsmodulen parallel und mit einem Minimum an Flüssigkeitsvolumen betrieben werden können. Das Perfusionsmodul, sowie Bioreaktoren umfassend solche Perfusionsmodule, eignen sich daher insbesondere für die Verwendung zum Studium des Verhaltens von Blutgefäßen, bzw. von Gefäßendothelzellen und/oder perivaskulären Glattmuskelzellen unter bestimmten ggf. veränderbaren Bedingungen, so z. B. zur Erforschung von physiologischen Vorgängen, wie beispielsweise der Angiogenese oder der Arteriogenese, und von Mechanismen bestimmter Erkrankungen, insbesondere gefäß-assoziierter Erkrankungen, zum Auffinden oder Testen von Wirkstoffen für die Behandlung von Erkrankungen oder für die *in vitro* Toxikologie.

Das erfindungsgemäße Perfusionsmodul umfasst eine oder mehrere Hohlfasern. Bevorzugt umfasst ein Perfusionsmodul eine Mehrzahl von Hohlfasern, die parallel zueinander angeordnet sind und betrieben werden können. Besonders bevorzugt umfasst ein Modul mehr als eine Hohlfaser, besonders bevorzugt 2 bis 80.000 Hohlfasern, besonders bevorzugt 2 bis 100 Hohlfasern. Jede Hohlfaser des Moduls umfasst oder besteht aus einer gas-und/oder flüssigkeitsdurchlässigen, semipermeablen Membran, die das Innenlumen der Hohlfaser von der Umgebung abgrenzt.

Die Membran weist eine durchschnittliche Porengröße von 0,01 bis 1 µm auf, damit Substanzen, wie Nährstoffe und/oder Wachstums- oder Botenstoffe frei durch die Membran diffundieren können. Bevorzugt weist die Membran eine durchschnittliche Porengröße von 0,002 bis 0,5µm auf, besonders bevorzugt von 0,002-0,04 oder 0,1 bis 0,3 µm, oder insbesondere von 0,2µm.

Um ein effektives "Anheften" der Endothelzellen und/oder Glattmuskelzellen zu ermöglichen, einen möglichst langen Co-Kulturzeitraum zu gewährleisten und/oder die Ausbildung eines für beide Zelltypen möglichst konfluenten Zellrasens zu erlauben, werden bevorzugt Hohlfasern eingesetzt, die eine Membran aufweisen, die ein Polyethersulfon(PES)-Polymer oder ein Polyethersulfon/Polyvinylpyrrolidon-Copolymer enthält oder daraus besteht.

Eine, mehrere oder alle Hohlfasern eines erfindungsgemäßen Moduls können einen Innendurchmesser von 100 µm bis 1000 µm aufweisen. Der Innendurchmesser ist somit groß genug, damit eine regel- und/oder steuerbare laminare Flüssigkeitsströmung im Lumen der Hohlfaser erzeugt und gehalten werden kann, so dass die Endothelzellen auf der luminalen Oberfläche der Hohlfaser einem bestimmten, vorher festlegbaren Scherstress ausgesetzt werden können. Auf der anderen Seite ist der Innendurchmesser klein genug, damit die Hohlfasern mit einem möglichst kleinen Flüssigkeitsvolumen betrieben werden können. Bevorzugt weist jede Hohlfaser des Moduls einen Innendurchmesser von 200 µm bis 900 µm, besonders bevorzugt von 500 µm bis 800 µm, insbesondere von 300 µm auf.

Jede Hohlfaser eines Moduls kann im Wesentlichen die gleiche luminale Oberfläche aufweisen, es können aber auch in einem Modul Hohlfasern vorgesehen sein, die unterschiedliche luminale Oberflächen aufweisen. Die Hohlfasern sind dabei derart ausgewählt und dimensioniert, dass die luminale Oberfläche eine Ausdehnung besitzt, die eine möglichst effektive Besiedelung mit Endothelzellen erlaubt. Bevorzugt weisen eine, mehrere oder alle Hohlfasern eines Moduls eine luminale Oberfläche auf von 1 bis 200 cm², besonders bevorzugt von 1 bis 100 cm², ganz besonders bevorzugt von 3 bis 10 cm², insbesondere von 7 cm².

Damit das Flüssigkeitsvolumen zur Kultur von Endothelzellen im Inneren der Hohlfasern möglichst gering bleibt, wird das luminale Volumen einer, mehrerer oder aller Hohlfasern eines Moduls möglichst klein gewählt. Bevorzugt umfasst das erfindungsgemäße Perfusionsmodul eine oder mehrere Hohlfasern, deren luminales Volumen jeweils nicht mehr als 1 L beträgt, bevorzugt nicht mehr als 100ml, besonders bevorzugt nicht mehr als 100 µl, ganz besonders bevorzugt von 10 bis 100 µl.

Um das Anheften von Zellen, beispielsweise von Endothelzellen und/oder von Glattmuskelzellen, an die luminale und/oder abluminale Oberfläche einer Hohlfaser des Moduls zu erleichtern oder zu gewährleisten, kann die luminale und/oder abluminale Oberfläche einer, mehrerer oder bevorzugt aller Hohlfasern eines Perfusionsmoduls mit einer Substanz beschichtet sein, die die Oberflächenadhäsion von Endothelzellen und/oder Glattmuskelzellen günstig beeinflusst oder befördert. Solche Substanzen sind dem Fachmann bekannt. Beispielhaft seien Fibronektin und/oder Collagene erwähnt. Insbesondere kann die luminale und/oder abluminale Oberfläche einer, mehrerer oder aller Hohlfasern eines Moduls mindestens teilweise mit Fibronektin beschichtet sein, bevorzugt sind sowohl die luminale als auch die abluminale Oberfläche der Hohlfaser mindestens teilweise mit Fibronektin beschichtet.

Im erfindungsgemäßen Perfusionsmodul sind mindestens Teile der luminalen Oberfläche der Hohlfasern mit Endothelzellen besiedelt, bevorzugt bilden die Endothelzellen auf dem entsprechenden Teil der luminalen Oberfläche einen konfluenten Zellrasen. Bei den Endothelzellen des Perfusionsmoduls kann es sich um primäre Endothelzellen handeln oder um Endothelzellen, die von immortalisierten Endothelzelllinien abstammen, die ggf. weitere genetische Veränderungen tragen können. Die primären Endothelzellen können von verschiedensten Blut- und/oder Lymphgefäßen stammen, so können die primären Endothelzellen aus Venen und/oder Arterien isoliert worden sein, aus Kapillaren und/oder aus größeren Gefäßen. Die Endothelzellen können ursprünglich aus Gefäßen bestimmter Organe isoliert worden sein, wie z. B. der Haut, der Nabelschnur, der quergestreiften und/oder der glatten Muskulatur, dem Herzen, dem Hirn, der Leber, dem Auge und/oder der Niere. Die Endothelzellen können grundsätzlich aus verschiedensten Spezies isoliert worden sein oder von solchen Zellen abstammen. Beispielhaft sei erwähnt, dass Endothelzellen von Rindern, Nagern, wie z. B. Ratten und/oder Mäusen, Schweinen, Kaninchen und/oder Menschen eingesetzt werden können. Bevorzugt werden Endothelzellen humanen Ursprungs verwendet. Die Endothelzellen können grundsätzlich aus jeder Phase der Entwicklung stammen, insbesondere können Endothelzellen aus embryonalen und/oder adulten Geweben verwendet werden. Besonders bevorzugt werden primäre HUVEC (human umbilical vein endothelial cells), HUAEC (human umbilical artery endothelial cells), HDMEC (human dermal microvascular endothelial cells) und/oder HAoEC (human aortic endothelial cells) verwendet. Dabei können im erfindungsgemäßen Perfusionsmodul auch Mischungen unterschiedlicher Endothelzelltypen eingesetzt werden.

Um den Endothelzellen Bedingungen bereitzustellen, unter denen diese möglichst lange kultiviert werden können, werden die Endothelzellen in einer Zelldichte auf der luminalen Oberfläche angesiedelt, bei der die Endothelzellen mindestens 48h überleben und/oder proliferieren können. Bevorzugt ist die luminale Oberfläche der Hohlfasern mindestens teilweise mit Endothelzellen in einer Dichte von nicht weniger als 0,5 x 10⁵ Zellen/cm² besiedelt, besonders bevorzugt von 1x10⁵ bis 3x10⁵, insbesondere von 2x10⁵ Zellen/cm².

Im erfindungsgemäßen Perfusionsmodul sind mindestens Teile der abluminalen Oberfläche der Hohlfasern mit Glattmuskelzellen besiedelt, bevorzugt bilden die Glattmuskelzellen auf dem entsprechenden Teil der abluminalen Oberfläche einen konfluenten Zellrasen. Bei den Glattmuskelzellen des Perfusionsmoduls kann es sich um primäre Glattmuskelzellen handeln oder um Glattmuskelzellen, die von immortalisierten Glattmuskelzelllinien abstammen, die ggf. weitere genetische Veränderungen tragen. Die primären Glattmuskelzellen können von verschiedensten Quellen, wie beispielsweise Blut- und/oder Lymphgefäßen stammen, so können die primären Glattmuskelzellen aus Venen und/oder Arterien isoliert worden sein, aus Kapillaren und/oder aus größeren Gefäßen. Die Glattmuskelzellen können ursprünglich aus Gefäßen bestimmter Organe isoliert worden sein, wie z. B. der Haut, der Nabelschnur, der quergestreiften und/oder der glatten Muskulatur, dem Herzen, dem Hirn, der Leber, dem Auge und/oder der Niere. Die Glattmuskelzellen können grundsätzlich aus verschiedensten Spezies isoliert worden sein oder von solchen Zellen abstammen. Beispielhaft sei erwähnt, dass Glattmuskelzellen von Rindern, Nagern, wie z. B. Ratten und/oder Mäusen, Schweinen, Kaninchen und/oder Menschen eingesetzt werden können. Bevorzugt werden Glattmuskelzellen humanen Ursprungs verwendet. Die Glattmuskelzellen können grundsätzlich aus jeder Phase der Entwicklung stammen, insbesondere können Glattmuskelzellen aus embryonalen und/oder adulten Geweben verwendet werden. Besonders bevorzugt werden primäre Glattmuskelzellen, insbesondere HUASMC (human umbilical artery smooth muscle cells), verwendet. Dabei können im erfindungsgemäßen Perfusionsmodul auch Mischungen unterschiedlicher Glattmuskelzelltypen eingesetzt werden.

Um den Glattmuskelzellen Bedingungen bereitzustellen, unter denen diese möglichst lange kultiviert werden können, werden die Glattmuskelzellen in einer Zelldichte auf der abluminalen Oberfläche angesiedelt, bei der die Glattmuskelzellen mindestens 48h überleben und/oder proliferieren können. Bevorzugt ist die abluminale Oberfläche der Hohlfasern mindestens teilweise mit Glattmuskelzellen in einer Dichte von nicht weniger als 0,5 x 10⁵ Zellen/cm² besiedelt, besonders bevorzugt von 1x10⁵ bis 3x10⁵, insbesondere von 2x10⁵ Zellen/cm².

Das erfindungsgemäße Perfusionsmodul weist zusätzlich zu den oben beschriebenen Hohlfasern, ein Gehäuse zur Aufnahme der Hohlfasern auf, sowie mindestens vier Zugänge. Dabei sind die Hohlfasern, das Gehäuse und die Zugänge eines Moduls derart ausgestaltet und angeordnet, dass der extraluminale und der intraluminale Raum der Hohlfasern eines Perfusionsmoduls getrennt voneinander regel- und/oder steuerbar mit Flüssigkeit perfundierbar sind. Eine Verbindung oder ein Übertritt zwischen extraluminalem und intraluminalem Raum eines Moduls ist bevorzugt ausschließlich über die oben beschriebene semipermeable Membran möglich. Die vier Zugänge sind dabei derart angeordnet, dass der intraluminale Raum eines Moduls über zwei Zugänge, z. B. einen Einlass und einen Auslass, regel- und/oder steuerbar perfundierbar ist, während der extraluminale Raum eines Moduls ebenfalls über zwei Zugänge, z. B. einen Einlass und einen Auslass, regel- und/oder steuerbar perfundierbar ist. Dabei ist keiner der zwei Zugänge für den extraluminalen Raum gleichzeitig einer der zwei Zugänge für den intraluminalen Raum eines Moduls. Über diese vier Zugänge ist es möglich den intraluminalen Raum und den extraluminalen Raum mit unterschiedlichem Kulturmedium zu versehen. Es ist auch möglich das konditionierte Medium des intraluminalen bzw des extraluminalen Raumes getrennt voneinander aus dem Modul abzuführen und ggf. zu sammeln. Die vier Zugänge erlauben es auch, die Endothelzellen auf der luminalen Oberflächhe der Hohlfasern einem bestimmten, vorher festgelegten Scherstress auszusetzen, während die Glattmuskelzellen auf der abluminalen Seite der Hohlfaser einem anderen vorher festgelegten Scherstress ausgesetzt werden können. Damit lässt sich die Situation in einem physiologischen Gefäß besonders gut nachstellen. Im erfindungsgemäßen Perfusionsmodul ist es also möglich insbesondere die Endothelzellschicht einer laminaren Strömung und damit einem bestimmten ggf. erhöhten Scherstress auszusetzen, der dem durch den Blutfluss vermittelten Scherstress entspricht. Dagegen können die Glattmuskelzellen auf der ablumminalen Seite, ebenso wie die perivaskulären Glattmuskelzellen eines Blutgefäßes, unter Bedingungen kultiviert werden, bei denen kein nennenswerter Scherstress entsteht.

Die vier Zugänge des erfindungsgemäßen Perfusionsmoduls können derart ausgestaltet sein, dass sie mit einem Flüssigkeitsreservoir und/oder einer Pumpe flüssigkeitsdicht verbindbar sind.

Die vorliegende Erfindung bezieht sich auch auf einen Bioreaktor umfassend ein oder mehrere erfindungsgemäße Perfusionsmodule. Dabei sind die Perfusionsmodule im erfindungsgemäßen Bioreaktor derart angeordnet, dass die einzelnen Perfusionsmodule parallel regel- und/oder steuerbar mit Flüssigkeit durchströmbar angeordnet sind.

Zusätzlich umfasst der Bioreaktor mindestens ein Flüssigkeitsreservoir. Das Flüssigkeitsreservoir kann insbesondere Kulturmedium für die Endothelzellen und/oder Glattmuskelzellen des oder der Perfusionsmodule des Bioreaktors enthalten. Werden der intraluminale Raum mit den Endothelzellen und der extraluminale Raum mit den Glattmuskelzellen mit dem selben Kulturmedium beschickt, werden also Endothelzellen und Glattmuskelzellen mit dem selben Medium kultiviert, so kann es sinnvoll sein, im erfindungsgemäßen Bioreaktor nur ein gemeinsames Flüssigkeitsreservoir vorzusehen, aus dem dann sowohl der extraluminale Raum als auch der intraluminale Raum eines, mehrerer oder aller Module des Bioreaktors beschickt werden. Der Biorekator kann aber auch zwei oder mehrere Flüssigkeitsreservoirs aufweisen.

Der erfindungsgemäße Bioreaktor umfasst mindestens einen Gasaustauscher. Dabei ist der Gasaustauscher derart ausgestaltet und angeordnet, dass Flüssigkeit aus dem Flüssigkeitsreservoir mit einer vorher festgelegten Menge an Gas, beispielsweise an O₂ und/oder CO₂, versehen werden kann. Bevorzugt wird der Gasgehalt der Flüssigkeit aus dem Flüssigkeitsreservoir angepasst und eingestellt, bevor die Flüssigkeit in die Perfusionsmodule gelangt. Sinn und Zweck ist es, den in den Perfusionsmodulen vorhandenen Endothelzellen und Glattmuskelzellen Bedingungen bereitzustellen, unter denen die Zellen überleben und ggf. proliferieren können.

Des weiteren weist der Bioreaktor eine Pumpe auf, die derart mit dem Flüssigkeitsreservoir und den Perfusionsmodulen verbunden ist, dass ein vorher festlegbarer Flüssigkeitsstrom vom Flüssigkeitsreservoir durch die Perfusionsmodule erzeugbar und aufrechterhaltbar ist. Bevorzugt ist die Pumpe derart ausgewählt, dimensioniert und angeordnet, dass sich im intraluminalen Raum der Perfusionsmodule Scherkräfte einstellen von 0,1 bis 50 dyne/cm². Bevorzugt werden Pumpen, z. B. Peristaltikpumpen, verwendet, die im Bereich von 0,1 ml bis 10 ml möglichst genau dosieren. Damit lassen sich Wandscherraten von 1 dyne/cm² bis 30 dyne/cm² einstellen und halten ohne die Viskosität erhöhende Flüssigkeitszusätze einsetzen zu müssen.

Zusätzlich kann der erfindungsgemäße Bioreaktor eine Steuer- und/oder Regeleinheit umfassen. Diese Steuer- und/oder Regeleinheit kann derart ausgestaltet und angeordnet sein, dass darüber der Betrieb des Bioreaktors gesteuert und/oder geregelt werden kann, insbesondere können darüber Flussgeschwindigkeiten, Scherraten und/oder Gaszusammensetzungen im Bioreaktor beeinflusst werden.

Die vorliegende Erfindung bezieht sich auch auf ein Verfahren zur Bestimmung von Veränderungen im Sekretom von Endothelzellen und/oder Glattmuskelzellen. Dabei wird unter einem Sekretom die Zusammensetzung aller von Endothelzellen und/oder Glattmuskelzellen an die Umgebung abgegebenen Stoffe verstanden. Insbesondere umfasst das Sekretom im Kulturmedium lösliche chemische Verbindungen, wie beispielsweise Proteine, Peptide, Nukleinsäuren, Zucker und/oder andere chemische Substanzen. Eine Veränderung im Sekretom bedeutet nicht nur die An- oder Abwesenheit eines bestimmten Stoffes oder einer bestimmten Verbindung, sondern umfasst auch Verschiebungen in der Gesamtzusammensetzung aller abgegebener Stoffe, so z. B. die verstärkte oder verminderte Präsenz eines bestimmten Stoffes oder einer bestimmten Verbindung.

Das erfindungsgemäße Verfahren umfasst die Schritte:
- Co-Kultur von Endothel- und Glattmuskelzellen mittels eines erfindungsgemäßen Perfusionsmoduls unter vorher festgelegten Kulturbedingungen;
- Sammeln des aus dem Perfusionsmodul abgeführten konditionierten Kulturmediums;
- Vergleich des konditionierten Kulturmediums mit einer Referenz.

Das erfindungsgemäße Verfahren eignet sich besonders gut zur Bestimmung von Veränderungen im Sekretom von Endothelzellen und/oder Glattmuskelzellen, da mittels der erfindungsgemäßen Perfusionsmodule eine Co-Kultur von Endothelzellen und Glattmuskelzellen über einen langen Zeitraum möglich ist. So können z. B. Co-Kulturzeiträume von 8 Tagen und mehr erreicht werden. Dadurch können einerseits die Zellen im Perfusionsmodul über einen langen Zeitraum unterschiedlichsten Kulturbedingungen ausgesetzt werden. Andererseits erlaubt das erfindungsgemäße Verfahren die Sammlung von Endothel- und/oder Glattmuskelzellen abgegeben Stoffen über einen langen Zeitraum, so dass insgesamt höhere Mengen an den sezernierten Stoffen für eine anschließende Analyse oder Weiterverwertung zur Verfügung stehen. So können mit dem vorliegenden Verfahren auch Stoffe nachgewiesen werden, die bei kürzerer Co-Kulturdauer nicht in ausreichendem Maße gewonnen werden können und somit bislang unterhalb einer Nachweisgrenze und damit unentdeckt geblieben sind.

Das erfindungsgemäße Verfahren umfasst die Co-Kultur von Endothelzellen und Glattmuskelzellen in einem erfindungsgemäßen Perfusionsmodul unter vorher festgelegten Kulturbedingungen.

Solche Kulturbedingungen können die Zusammensetzung des Mediums betreffen. Bevorzugt erfolgt die Co-Kultur unter einem gemeinsamen Kulturmedium, welches sowohl für die Kultur von Glattmuskelzellen im abluminalen Raum als auch für die Kultur von Endothelzellen im intraluminalen Raum des Perfusionsmoduls verwendet wird. Besonders bevorzugt erfolgt die Co-Kultur unter folgendem Medium: Smooth Muscle Cell Growth Medium 2 (Fa. PromoCell Cat.: C-22062 + Endothelial Cell Growth Supplement/Heparin (Fa. PromoCell, Cat.: C-30120).

Dem Kulturmedium können zusätzliche Stoffe zugesetzt werden. Solche Stoffe können Verbindungen umfassen, deren Einfluss auf die Endothel- und/oder Glattmuskelzellen der Co-Kultur bzw. auf deren Sekretom untersucht werden soll.

Die Kulturbedingungen können auch die Gaszusammensetzung des Kulturmediums betreffen. So kann beispielsweise untersucht werden, wie Endothelzellen und/oder Glattmuskelzellen auf Hypoxie oder Hyperoxie reagieren. Dazu kann beispielsweise der Gehalt an O₂ und/oder CO₂ im Kulturmedium entsprechend variiert werden.

Die Kulturbedingungen umfassen auch die Kulturdauer. Das vorliegende Verfahren zeichnet sich insbesondere dadurch aus, dass sehr lange Co-Kulturzeiträume möglich sind. Bevorzugt können Co-Kulturzeiträume von mehr als 8 Tagen realisiert werden.

Die Kulturbedingungen umfassen beispielsweise auch bestimmte ausgewählte Scherstressraten, die an die Endothelzell-tragenden Innenoberflächen der Hohlfasern angelegt werden können, unter denen dann die Co-Kultur erfolgt.

Grundsätzlich hängen die Kulturbedingungen von der beabsichtigten Fragestellung ab. So können die Kulturbedingungen so gewählt werden, dass Bedingungen eingestellt werden, wie sie bei bestimmten physiologischen Vorgängen im Körper, bei Erkrankungen oder krankhaften Zuständen oder Veränderungen, insbesondere bei Erkrankungen betreffend das Gefäßbett, herrschen oder vorkommen können. Es können dann die Reaktionen der Endothelzellen und/oder der Glattmuskelzellen auf solche Bedingungen untersucht werden.

Aus dem Perfusionsmodul wird das konditionierte Kulturmedium, enthaltend die von den Zellen abgegebenen Stoffe und Verbindungen, abgeführt, aufgefangen und/oder gesammelt. Die das Sekretom bildenden, von den Endothelzellen und/oder den Glattmuskelzellen des Perfusionsmoduls freigesetzten Stoffe und Verbindungen befinden sich nun im konditionierten Kulturmedium und können einer weiteren Analyse oder Weiterbehandlung zugeführt werden. Dazu kann das gesammelte konditionierte Kulturmedium weiteren Behandlungsschritten unterzogen werden, bei denen beispielsweise bestimmte Stoffe aus dem konditionierten Kulturmedium entfernt oder isoliert werden oder bestimmte Stoffe oder Stoffgruppen angereichert werden, so dass im weiteren Verfahren ggf. nur noch Bestandteile oder Fraktionen des konditionierten Kulturmediums betrachtet werden.

Das isolierte und ggf. weiteren Behandlungsschritten unterzogene konditionierte Kulturmedium wird dann mit einer Referenz verglichen, um ggf. eine Abweichung festzustellen. Dabei kann zunächst die Anwesenheit und/oder Abwesenheit von Stoffen oder Verbindungen untersucht werden. Es können aber auch Verschiebungen in der Häufigkeit bestimmter ausgewählter Stoffe oder Verbindungen betrachtet und festgestellt werden. Beispielsweise kann das Sekretom einer Endothelzell- und Glattmuskelzell-Co-Kultur unter hypoxischen, hyperoxischen oder bestimmten Scherstressbedingungen mit dem Sekretom einer Endothelzell- und Glattmuskelzell-Co-Kultur unter normoxischen Bedingungen und/oder anderen Scherstressbedingungen verglichen werden.

Eine Möglichkeit der Bestimmung und des Vergleichs von Sekretomen ist die Aufnahme von MALDI-TOF/TOF MS-Spektren der Sekretome bzw. der konditionierten Kulturmedien oder ggf. Bestandteilen davon und der Vergleich der Spektren unterschiedlicher Kultur- oder Behandlungsbedingungen. Dabei bildet ein erstes Spektrum das Referenzspektrum, während anhand des Referenzspektrums Abweichungen und Veränderungen in einem zweiten Spektrum festgestellt und ggf. quantifiziert werden können.

Die vorliegende Erfindung bezieht sich auch auf eine Verwendung eines erfindungsgemäßen Perfusionsmodul, eines erfindungsgemäßen Bioreaktors und/oder eines erfindungsgemäßen Verfahrens zur Bestimmung von Auswirkungen vorher festgelegter Bedingungen auf das Gefäßbett.

Das erfindungsgemäße Perfusionsmodul, der erfindungsgemäße Bioreaktor und/oder das erfindungsgemäße Verfahren kann auch zum Auffinden oder Testen von Wirkstoffen für die Beeinflussung von physiologischen Prozessen wie z. B. Arteriogenese, Angiogenese, und/oder zur Behandlung von Erkrankungen, bevorzugt von Herz-Kreislauferkrankungen, Krebs, Hypercholesterinämie, Hypertonie, Diabetes mellitus, Adipositas, Atherosklerose, Calzifizierung, endotheliale Dysfunktion, chronische Niereninsuffizienz und/oder metabolisches Syndrom verwendet werden.

Insbesondere bei der Wirkstoffentwicklung für Medikamente oder für sonstige Wirkstoffe, die in der Umwelt freigesetzt werden sollen, ist es häufig erforderlich toxikologische Studien durchzuführen, bevor solche Stoffe oder diese Stoffe enthaltende Produkte auf den Markt gebracht und verkauft werden dürfen. Ein Problem bei solchen toxikologischen Untersuchungen ist, dass solche Untersuchungen häufig nur in Versuchstieren durchgeführt werden können, die den später mit den Stoffen konfrontierten Organismen nicht in allen Belangen entsprechen. Im Falle von Medikamenten für den humanmedizinischen Gebrauch werden solche toxikologischen Studien meist an Mäusen und/oder Ratten durchgeführt. Der Metabolismus dieser Versuchstiere unterscheidet sich allerdings von dem der späteren Nutzer des Produktes, so dass die Aussagen manchmal nur bedingt übertragbar sind. Insbesondere für Organe, die mit der betreffenden Substanz oder Wirkstoff erst nach einer Aufnahme und ggf. metabolischen Veränderung in Kontakt gelangen, sind die Ergebnisse in Versuchstieren problematisch. Das Gefäßsystem stellt ein solches Organ dar, bei dem es wünschenswert wäre einerseits die Versuchstiere zu schonen und andererseits einen Test einzusetzen, der der tatsächlichen Situation beispielsweise im Menschen näher kommt.

Insbesondere des erfindungsgemäße Perfusionsmodul, der erfindungsgemäße Bioreaktor und/oder das erfindungsgemäße Verfahren eignen sich für eine Verwendung in der *in vitro* Toxikologie und haben das Potential Tierversuche zu ersetzen. Bevorzugt werden dabei Endothelzellen und/oder Glattmuskelzellen humanen Ursprungs eingesetzt.

### Figuren:

- Figur 1: zeigt eine Darstellung der Besiedelung einer Hohlfaser mit glatten Gefäßmuskelzellen (A und C) auf der abluminalen Oberfläche der Hohlfaser und der Besiedelung einer Hohlfaser mit Endothelzellen (B und D) auf der luminalen Oberfläche der Hohlfaser.
- Figur 2: zeigt eine Raster-Elektronen-Aufnahme (REM) von HUVEC nach Besiedelung einer luminalen Oberfläche eines Hohlfasermoduls.
- Figur 3: zeigt eine schematische Darstellung des Aufreinigungsschema zur Quantifizierung des Up₄A aus endothelialen Sekretomen.
- Figur 4: zeigt eine charakteristische chromatographische Aufreinigung eines endothelialen Sekretoms. **(A)** präparatives Reversed-Phase-Chromatogramm; **(B)** Affinitätschromatogramm; **(C)** analytische Reversed-Phase-Chromatogramm. Die Up₄A-enthaltenden Fraktionen sind jeweils mit einem Pfeil markiert.
- Figur 5: zeigt MALDI-TOF/TOF-Massenspektren des Sekretoms von Endothelzellen, die **(A)** mit der in Jankowski V et al. (Nature Medicine. 2005;11(2):223-227) beschriebenen Methode und **(B)** mittels des Bioreaktor stimuliert wurden.

### Beispiele:

### Beispiel 1: Aufbau und Betrieb eines erfindungsgemäßen Bioreaktors

Module wurden mit 70%igem Ethanol hydrophiliert, indem die Ethanollösung mit Hilfe von Spritzen von der intrakapillaren Seite (IC) zur extrakapillaren Seite (EC) hin und zurück gespült wurde. Nach dem Ausspülen des Ethanols mit Wasser wurde das Modul mit dem sterilen Schlauschystem (inkl. Reservoir) verbunden und mit PBS (mit Ca/Mg) für mindestens 48 h bei 37 °C unter geringem PBS-Fluß equilibriert. Vor der Zellbesiedlung wurden das Modul mit Fibronektin (10 µg/ml) für 1,5 h bei 37°C unter statischen Bedingungen beschichtet und anschließend unter geringem Fluss mit Kokulturmedium für mind.1 h equilibriert. Die Hohlfasern wurden auf der Innenseite mit Endothelzellen (HUVECs) besiedelt mit einer Zelldichte von 200 000 Zellen /cm². Hierfür wurde die Zellsuspension von der IC-Seite zur EC-Seite filtriert. Nach einer Inkubationszeit von 1 h unter statischen Bedingungen wurden die Zellen anschließend über den Extrakapillarraum für 2 h mit Kokulturmedium (20 ml) unter geringem Mediumfluss versorgt. Die Außenseite der Hohlfaserkapillaren wurde mit glatten Muskelzellen (HUASMCs) mit einer Zelldichte von 200 000 Zellen/cm² besiedelt, indem die Zellsuspension von der EC-Seite zur IC-Seite hin filtriert wurde. Nach einer Adhärierungsphase von 1,5 h bei 37 °C startete die Ausübung von Scherstress von 1 dyne/cm² (Kontrolle) im Vergleich zu 30 dyne/cm². Die Höhe der Scherrrate wurde entsprechend über die Höhe des Mediumflusses gesteuert. Bei Langzeitexperimenten erfolgte jeden Tag ein Mediumwechsel (20 ml) (Smooth Muscle Cell Growth Medium 2 (Fa. PromoCell Cat.: C-22062 + Endothelial Cell Growth Supplement/Heparin (Fa. PromoCell, Cat.: C-30120))

Die Module, bestehend aus 14 Plasmaphan P1LX-Hohlfasern (Polypropylen) mit einer effektiven Innenfläche von 7 cm², einem Durchmesser von 330 µM und einer Wandstärke von 150 µM waren über vier Zugänge mit einem Schlauchsystem verbunden, welches eine parallele Versorgung des IC- und EC-Raumes mit Medium ermöglichte. Ein Mediumreservoir mit einem max. Fassungsvermögen von 125 ml diente zur Rezirkulation des Mediums und ermöglichte durch einen Luftfilteraufsatz einen optimalen Gasaustausch im Inkubator.

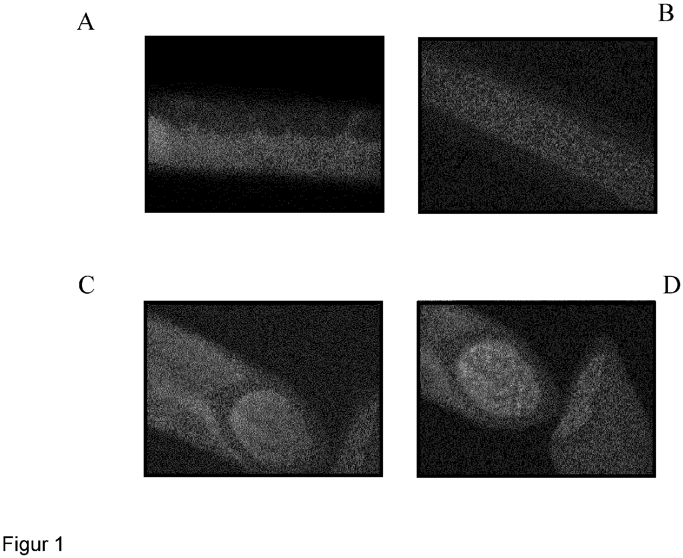

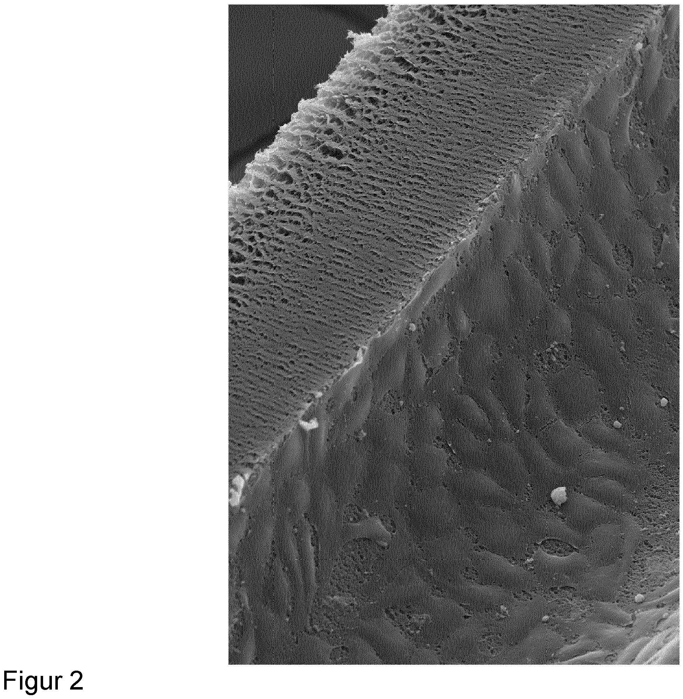

Die erfolgreiche Besiedelung der Hohlfaser ist in Figuren 1 und 2 gezeigt.

### Beispiel 2: Auswertung am Beispiel eines Sekretoms bzw. eines interessanten Faktors aus dem Sekretom

Es konnte gezeigt werden, dass eine hohe Reproduzierbarkeit der Ergebnisse durch die Aufreinigung des jeweiligen deproteinierten Sekretoms mittels präparativer Reversed-Phase-Chromatographie und Micro-Reversed-Phase-Chromatographie erzielt werden kann.

MALDI-TOF/TOF-massenspektrometrische Fingerprint-Analysen können verwendet werden, um die Zusammensetzung endothelial sezernierter Mediatoren zu bestimmen. Die resultierenden MALDI-TOF/TOF-Massenspektren können sowohl hinsichtlich qualitativer als auch hinsichtlich quantitativer Aspekte ausgewertet werden. Durch diesen MALDI-TOF/TOFmassenspektrometrischen Ansatz ist es möglich, sowohl qualitative als auch quantitative Daten von den jeweiligen Sekretomen zu generieren, ohne dass die Identität der jeweiligen Mediatoren vorab geklärt werden muß. Durch diesen Ansatz können sowohl quantitative als auch qualitative Veränderungen sehr effektiv und mit hoher Validität bestimmt werden.

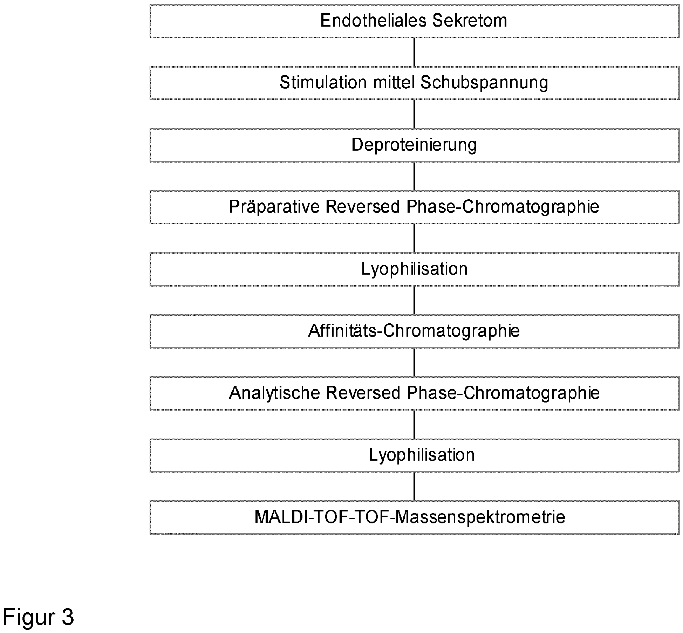

Das Uridin-Adenosin-Tetraphosphat (Up₄A) ist ein bedeutsamer Indikator einer ungestörten endothelialen Funktion (Jankowski V. et al., Arterioscler Thromb Vasc Biol. 2007;27(8):1776-1781., Jankowski V et al., Nature Medicine. 2005;11(2):223-227.). Daher wurde dieser Faktor im Rahmen der quantitativen Analyse zur Bestimmung des "Proof of concept" der Modelläquivalenz eingesetzt. Hierzu wurde ein Assay etabliert, durch den Up₄A im endothelialem Sekretom mit hoher Validität zu quantifizieren ist. Der Assay basiert auf chromatographische Methoden, die aufgrund der molekularen Struktur des Up₄A ausgewählt worden waren. **Figur 3** zeigt das im Rahmen des Projekts etablierte Aufreinigungsschema zur Quantifizierung des Up₄A aus endothelialen Sekretomen.

Die endothelialen Sekretome wurden deproteiniert, mittels einer präparativen Reversed-Phase-Chromatographie konzentriert und fraktioniert. Die Eluate der präparativen Reversed-Phase-Chromatographie wurden lyophilisiert und anschließend mittel einer Affinitätschromatographie weiter fraktioniert. Das Eluat der Affinitätschromatographie wurde mit einer analytischen Reversed-Phase-Chromatographie konzentriert und entsalzt. Der Up₄A-Gehalt im Eluat der Reversed-Phase-Chromatographie wurde mittels der MALDI-TOF/TOF-Massenspektrometrie quantifiziert.

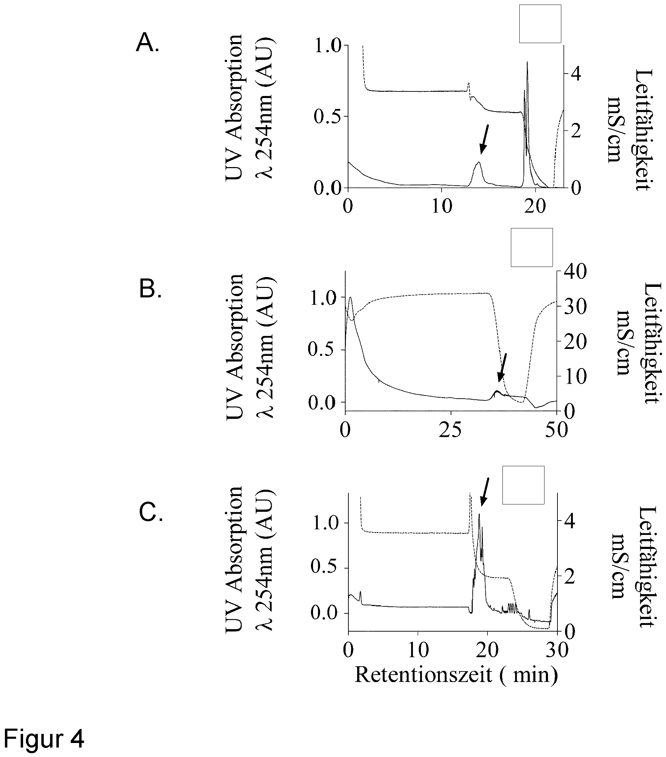

**Figur 4** zeigt beispielhaft die chromatographische Aufreinigung eines endothelialen Sekretoms. Das entsprechende endotheliale Sekretom wurde nach Stimulation mit 30 dyn für 24 h unter Verwendung des Plattenkegelmodels erhalten. In **Figur 4A** ist ein Chromatogramm der präparativen Reversed-Phase-Chromatographie des endothelialen Sekretoms gezeigt. Durch Referenzchromatographien authentischen Up₄A konnten die Fraktionen identifiziert werden, die Up₄A enthielten. Die entsprechenden Fraktionen sind in **Figur 4A** markiert. **Figur 4B** zeigt ein charakteristisches Affinitätschromatogramm der Reversed-Phase-Chromatographie-Fraktion, die in **Figur 4A** mit einem Pfeil markiert worden ist. Ein analytisches Reversed-Phase-Chromatogramm der in **Figur 4B** markierten Fraktion zeigt **Figur 4C****.** Durch Referenzchromatographien konnte gezeigt werden, dass die in **Figur 4C** markierte Fraktionen Up₄A enthalten. Die entsprechenden Fraktionen wurden der MALDI-TOF/TOF-Massenspektrometrie zugeführt.

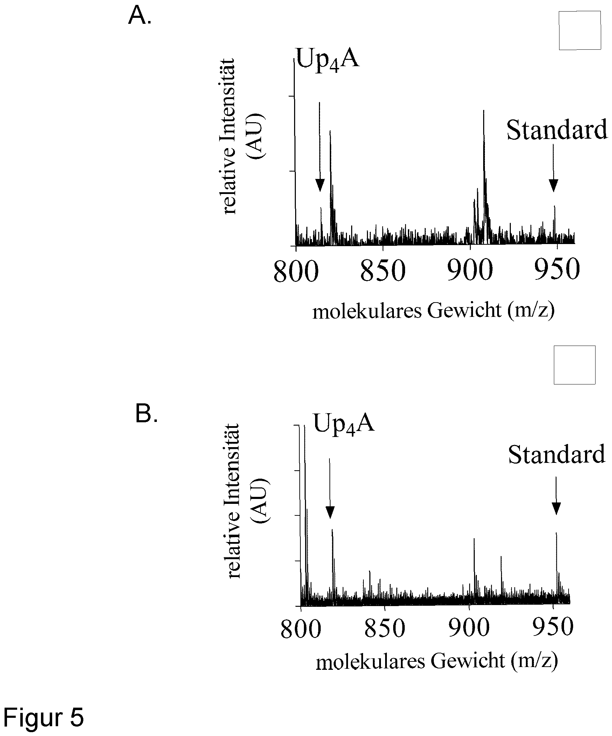

Weiterhin wurde Up₄A im Sekretom von Endothelzellen bestimmt, die (Figur 5A) mit der in Jankowski V et al (Nature Medicine. 2005;11(2):223-227) beschriebenen Methode und (Figur 5B) mittels eines Plattenkegel-Modells stimuliert worden waren. Die jeweiligen endothelialen Sekretome wurden mittels des im Rahmen des Projekts etablierten Assays chromatographisch fraktioniert. Die resultierenden MALDI-TOF/TOF-Massenspektren sind in Figur 5A beziehungsweise Figur 5B gezeigt. Die Ergebnisse dieser Bestimmungen zeigen, dass der Up₄A-Gehalt in den entsprechenden Sekretomen in identischen Größenordnungen vorliegt.

## Patentansprüche

1. Perfusionsmodul, umfassend:
i) eine oder mehrere Hohlfasern umfassend oder bestehend aus einer gas-und/oder flüssigkeitsdurchlässigen Membran mit einer durchschnittlichen Porengröße von 0,002 bis 1 µm, wobei mindestens Teile der luminalen Oberfläche der Hohlfasern mit Endothelzellen und mindestens Teile der abluminalen Oberfläche der Hohlfasern mit Glattmuskelzellen besiedelt sind;
ii) ein Gehäuse zur Aufnahme der Hohlfasern; und
iii) mindestens vier Zugänge,
wobei die Hohlfasern, das Gehäuse und die Zugänge derart ausgestaltet und angeordnet sind, dass der extraluminale und der intraluminale Raum der Hohlfasern eines Perfusionsmoduls getrennt voneinander regel- und/oder steuerbar mit Flüssigkeit perfundierbar sind.

2. Perfusionsmodul nach Anspruch 2, **dadurch gekennzeichnet, dass** eine, mehrere oder alle Hohlfasern des Moduls eine Membran aufweisen, die ein Polyethersulfon(PES)-Polymer oder ein Polyethersulfon/Polyvinylpyrrolidon-Copolymer enthält oder daraus besteht.

3. Perfusionsmodul nach einem der vorhergehenden Ansprüche, **dadurch gekennzeichnet, dass** eine, mehrere oder alle Hohlfasern des Moduls einen Innendurchmesser von 100 µm bis 1000 µm aufweisen.

4. Perfusionsmodul nach einem der vorhergehenden Ansprüche, **dadurch gekennzeichnet, dass** eine, mehrere oder alle Hohlfasern eine luminale Oberfläche aufweisen von 1 cm² bis 20 m².

5. Perfusionsmodul nach einem der vorhergehenden Ansprüche, **dadurch gekennzeichnet, dass** das luminale Volumen einer, mehrerer oder aller Hohlfasern eines Moduls jeweils nicht mehr als 1 L beträgt.

6. Perfusionsmodul nach einem der vorhergehenden Ansprüche, **dadurch gekennzeichnet, dass** die luminale und/oder die abluminale Oberfläche der Hohlfasern mindestens teilweise mit Fibronectin beschichtet ist.

7. Perfusionsmodul nach einem der vorhergehenden Ansprüche, **dadurch gekennzeichnet, dass** die luminale Oberfläche der Hohlfasern mindestens teilweise mit Endothelzellen in einer Dichte von nicht weniger als 0,5 x 10⁵ Zellen/cm² besiedelt ist.

8. Perfusionsmodul nach einem der vorhergehenden Ansprüche, **dadurch gekennzeichnet, dass** die abluminale Oberfläche der Hohlfasern mindestens teilweise mit Glattmuskelzellen in einer Dichte von nicht weniger als 0,5 x 10⁵ Zellen/cm² besiedelt ist.

9. Bioreaktor umfassend
i) eine Mehrzahl von Perfusionsmodulen gemäß einem der Ansprüche 1 bis 8, wobei die einzelnen Perfusionsmodule parallel mit Flüssigkeit durchströmbar angeordnet sind;
ii) ein Flüssigkeitsreservoir;
iii) einen Gasaustauscher, der derart angeordnet ist, dass Flüssigkeit aus dem Flüssigkeitsreservoir mit einer vorher festgelegten Menge an Gas, insbesondere O₂ und/oder CO₂, versehen werden kann; und
iv) einer Pumpe, die derart mit dem Flüssigkeitsreservoir und den Perfusionsmodulen verbunden ist, dass ein vorher festlegbarer Flüssigkeitsstrom vom Flüssigkeitsreservoir durch die Perfusionsmodule aufrechterhaltbar ist.

10. Bioreaktor nach Anspruch 9 **dadurch gekennzeichnet, dass** der Bioreaktor zusätzlich eine Steuer- und/oder Regeleinheit umfasst.

11. Verfahren zur Bestimmung von Veränderungen im Sekretom von Endothelzellen und/oder Glattmuskelzellen, umfassend die Schritte:
- Co-Kultur von Endothel- und Glattmuskelzellen mittels eines Perfusionsmoduls nach einem der Ansprüche 1 bis 8 unter vorher festgelegten Kulturbedingungen;
- Sammeln des aus dem Perfusionsmodul abgeführten konditionierten Kulturmediums;
- Vergleich des konditionierten Kulturmediums mit einer Referenz.

12. Verfahren nach Anspruch 11 **dadurch gekennzeichnet, dass** vom abgeführten konditionierten Zellkulturmedium oder Teilen davon ein MALDI-TOF/TOF MS-Spektrum gewonnen wird und mit einem Referenzspektrum verglichen wird.

13. Verwendung eines Perfusionsmoduls nach einem der Ansprüche 1 bis 8, eines Bioreaktors nach einem der Ansprüche 9 oder 10 oder eines Verfahrens nach einem der Ansprüche 11 der 12 zur Bestimmung von Auswirkungen vorher festgelegter Bedingungen auf das Gefäßbett.

14. Verwendung eines Perfusionsmoduls nach einem der Ansprüche 1 bis 8, eines Bioreaktors nach einem der Ansprüche 9 oder 10 oder eines Verfahrens nach einem der Ansprüche 11 oder 12 zum Auffinden oder Testen von Wirkstoffen für die Behandlung von Erkrankungen, bevorzugt von Herz-Kreislauferkrankungen, Arteriogenes, Angiogenese, Krebs, Hypercholesterinämie, Hypertonie, Diabetes mellitus, Adipositas, Atherosklerose, Calzifizierung, endotheliale Dysfunktion, chronische Niereninsuffizienz, metabolisches Syndrom.

15. Verwendung eines Perfusionsmoduls nach einem der Ansprüche 1 bis 8, eines Bioreaktors nach einem der Ansprüche 9 oder 10 oder eines Verfahrens nach einem der Ansprüche 11 oder 12 für die in vitro Toxikologie.
